# EUROPÄISCHE PATENTANMELDUNG

(11) **EP 2 177 156 A1**
(43) Veröffentlichungstag der Anmeldung: **21.04.2010**
(21) Anmeldenummer: 09012937.0
(22) Anmeldetag: 13.10.2009
(51) Int. Cl.: A61B 5/024

(54) **Kleidungsstück zum Überwachen physiologischer Eigenschaften**

(30) Priorität: 14.10.2008 DE 102008051536
(71) Anmelder: Cairos technologies AG, 76307 Karlsbad (DE)
(72) Erfinder: Holzer, Christian, 81671 München (DE); Habel, Thorsten, 75045 Walzbachtal (DE); Gierich, Martin, 76297 Stutensee (DE)
(74) Vertreter: Grünecker, Kinkeldey, Stockmair & Schwanhäusser Anwaltssozietät

(57) **Zusammenfassung**

Ein Kleidungsstück ermöglicht das Überwachen physiologischer Eigenschaften eines Trägers des Kleidungsstücks. Dabei sind die Messsensoren in das Kleidungsstück integriert und werden durch Anlegen des Kleidungsstücks an die richtigen Stellen des Körpers des Trägers positioniert. Die Vorrichtung zum Überwachen der physiologischen Eigenschaften, die die Messelektronik enthält, befindet sich hierbei im Rückenteil des Kleidungsstücks. Diese Vorrichtung ist entweder fest in das Kleidungsstück integriert oder kann abgenommen werden.

## Beschreibung

Die vorliegende Erfindung ermöglicht die Messung physiologischer Eigenschaften einer Person, im Besonderen der Herzfrequenz während des Spielens einer Mannschafts- oder Ballsportart.

Für ein optimales und gesundes Fitness- und Ausdauertraining ist die Messung der Herzfrequenz während des Trainings von entscheidender Bedeutung. Während das manuelle Fühlen des Herzschlags ungenaue Ergebnisse liefert, sind jedoch herkömmliche Elektrokardiogramm (EKG)-Geräte für die Benutzung während des Sports zu kostenintensiv und zu komplex.

Das erste kabellose Herzfrequenz-Messgerät (auch bekannt als Pulsuhr oder Pulsmesser) war das tragbare PE 2000 von Polar Electro OY. Das PE 2000 Herzfrequenz-Messgerät besteht aus einem Empfänger und einem Sender, der entweder durch Einmal-Elektroden oder einen elastischen Elektrodengurt an der Brust angebracht wird. Der Empfänger ist ein uhrenähnlicher Monitor, der am Handgelenk getragen werden kann.

Die gängigen Herzfrequenz-Messgeräte nehmen über einem an der Brust getragenen Sender die Herzsignale (R-Impulse) auf, die über die Haut abgegeben werden, und senden diese Signale an den Empfänger, der am Handgelenk getragen oder an einem Fahrrad bzw. Sportgerät befestigt wird. Die aktuelle Herzfrequenz wird dann kontinuierlich im Display des Empfängers angezeigt.

Im Fitness-Bereich werden häufig stationäre Herzfrequenzmesser an den Geräten selbst eingesetzt, die die Herzfrequenz über Sensoren abnehmen, die für die Messung umfasst werden müssen. Andere Geräte messen die Herzfrequenz am Ohrläppchen. Für den leistungsorientierten Bereich sind beide letztgenannten Messmethoden jedoch nicht geeignet.

WO 07/040878 A1 offenbart eine Weiterentwicklung der herkömmlichen Herzfrequenz-Messgeräte. Hier werden die Elektroden zur Messung des Herzschlags in ein Kleidungsstück integriert. Hierbei befinden sich zwei Elektroden auf der Vorderseite des Kleidungsstücks und eine Elektrode auf der Rückseite. Des Weiteren ist eine Vorrichtung zur Verarbeitung der gemessenen Signale entweder im Kleidungsstück integriert oder kann beispielsweise mechanisch mittels Druckknöpfen am Kleidungsstück befestigt werden.

Ähnliches bietet Adidas in Zusammenarbeit mit Polar Electro an. Hier kann ein Herzfrequenzmessgerät direkt an der Brust auf einem T-Shirt befestigt werden. Auch dieses System macht einen Brustgurt überflüssig.

Allerdings sind diese herkömmlichen Systeme zur Messung der Herzfrequenz für Personen, die eine Mannschafts- oder Ballsportart ausüben, nur bedingt geeignet. So ist vor allem bei Ballsportarten der Brustbereich oder der gesamte Vorderbereich des Oberkörpers oft in das Spiel mit eingebunden. Fußballspieler stoppen beispielsweise eine Flanke mit der Brust. Ähnliches gilt für Basketballspieler, bei denen der Vorderbereich des Oberkörpers des Öfteren beim Annehmen oder Abspielen des Balles mit dem Ball in Berührung kommt. Demnach werden Sensoren im Brustbereich in solchen Fällen als störend empfunden. Mehr noch, Sensoren im Brustbereich sind durch auftreffende Bälle selbst sehr gefährdet. Befindet sich außerdem die Verarbeitungs- und/oder Sendeeinheit ebenfalls im Brustbereich, wie beim System von Adidas, so erhöht sich das Verletzungsrisiko des Spielers. Das Verletzungsrisiko erhöhen ebenfalls die in WO 07/040878 A1 vorgeschlagenen Druckverbindungen zur Ankopplung der Verarbeitungseinheit.

Demnach liegt die Aufgabe der vorliegenden Erfindung darin, das Überwachen physiologischer Eigenschaften von Personen zu ermöglichen, die gerade eine Mannschafts- oder Ballsportart ausüben. Diese Aufgabe wird erfindungsgemäß durch den Gegenstand des unabhängigen Patentanspruchs gelöst. Bevorzugte Ausführungsformen werden durch den Gegenstand der abhängigen Patentansprüche definiert.

Erfindungsgemäß umfasst ein Kleidungsstück eine Vorrichtung zum Überwachen physiologischer Eigenschaften eines Trägers des Kleidungsstücks. Diese Vorrichtung zum Überwachen ist auf der Rückseite des Kleidungsstücks positioniert.

Bevorzugte Ausgestaltungen der vorliegenden Erfindung werden im Weiteren mit Bezug auf folgende Zeichnungsfiguren im Detail beschrieben. Hierbei zeigen die Zeichnungen im Einzelnen:
- Fig. 1a: Seitenansicht eines Kleidungsstücks zur Überwachung physiologischer Eigenschaften gemäß einer Ausführungsform;
- Fig. 1b: Frontansicht eines Kleidungsstücks zur Überwachung physiologischer Eigenschaften gemäß einer Ausführungsform;
- Fig. 1c: Rückansicht eines Kleidungsstücks zur Überwachung physiologischer Eigenschaften gemäß einer Ausführungsform;
- Fig. 2a: Rückansicht eines Kleidungsstücks zur Überwachung physiologischer Eigenschaften gemäß einer Ausführungsform;
- Fig. 2b: Rückansicht eines Kleidungsstücks zur Überwachung physiologischer Eigenschaften gemäß einer Ausführungsform;
- Fig. 2c: Rückansicht eines Kleidungsstücks zur Überwachung physiologischer Eigenschaften gemäß einer Ausführungsform
- Fig. 3a: perspektivische Ansicht einer Vorrichtung zur Überwachung physiologischer Eigenschaften gemäß einer Ausführungsform;
- Fig. 3b: perspektivische Ansicht einer Vorrichtung zur Überwachung physiologischer Eigenschaften gemäß einer Ausführungsform; und
- Fig. 4: schematische Darstellung einer Vorrichtung zur Überwachung physiologischer Eigenschaften gemäß einer Ausführungsform.

Für optimale Trainingserfolge muss das Training auf die Physiologie des Sportlers abgestimmt werden. Hierzu ist eine Überwachung von physiologischen Eigenschaften der Sport treibenden Person während des Trainings unerlässlich. Wichtige physiologische Eigenschaften, die zur Optimierung des Trainings führen, aber auch aus medizinischer Sicht von Bedeutung sind, können hierbei die Herzfrequenz, die Atemfrequenz, die Sauerstoffsättigung des Blutes, die Durchblutung einzelner Organe, die Identifizierung der benutzten Muskelgruppen oder die Belastung der Skelettstruktur sein.

Zur Überwachung der physiologischen Eigenschaften sind eine Vielzahl von Messverfahren und Sensoren denkbar. So liefert die Aufzeichnung eines Elektrokardiogramms (EKG) aufschlussreiche Erkenntnisse über die Herztätigkeit während des Trainings. Ist man nur an der Herzfrequenz interessiert, so muss kein vollständiges EKG angefertigt werden, sondern nur der R-Impuls des Herzens ausgewertet werden.

Um die Physiologie einzelner Organe während des Trainings zu überwachen, ist es weiter denkbar, Sonogramme mit oder ohne Anwendung des Doppler-Verfahrens anzufertigen. So können mit Hilfe einer Doppler-Ultraschall-Sonografie Aussagen beispielsweise über den Blutfluss im Herzen während des Sports getroffen werden. Außerdem können Ultraschallmessungen während des Trainings Aufschlüsse über die Belastung der Skelettstruktur liefern.

Daneben ist der Einsatz anderer Sensoren, wie Infrarotsensoren zur Ermittlung besonders aktiver Regionen im Körper oder Bewegungssensoren zum Optimieren von Bewegungsabläufen möglich. Ebenfalls denkbar ist der Einsatz von radioaktiven Tracer-Substanzen, um die Durchflussgeschwindigkeit des Blutes zu messen. Hierbei wird die Ausbreitung einer im Blutkreislauf des Sportlers injizierten radioaktiven Tracer-Substanz mittels Messung der radioaktiven Aktivität ermittelt.

Die Sensoren, die Messelektronik und andere Verarbeitungseinheiten für die Überwachung der physiologischen Eigenschaften von Sportlern während des Trainings dürfen allerdings den normalen Bewegungsablauf des Sportlers nicht oder nur sehr gering stören, um von den Sportlern und Trainern akzeptiert zu werden. Des Weiteren würde die Messung physiologischer Eigenschaften bei gestörtem Bewegungsablauf nicht das normale Training optimieren, sondern das gestörte Training.

Vor allem bei Mannschafts- oder Ballsportarten gelten für die Überwachung von physiologischen Eigenschaften während des Ausübens der Sportart besondere Anforderungen an die ausführenden Vorrichtungen. So ist es nicht ungewöhnlich, dass der Brustbereich des Sportlers, im Besonderen bei Ballsportarten, häufig in Berührung mit einem Spielgerät oder mit einem anderen Spieler kommt. Hierbei besteht die Gefahr, dass die Sensoren zur Überwachung der physiologischen Eigenschaft, beispielsweise beim Auftreffen eines Balls, beschädigt werden. Außerdem empfinden die meisten Ball- oder Mannschaftssportler technisches Equipment im vorderen Bereich des Oberkörpers als störend. Schließlich erhöht sich durch Anbringen von Messinstrumenten an der Vorderseite des Oberkörpers die Verletzungsgefahr.

Gemäß einer Ausführungsform der Erfindung wird demnach das komplette Messequipment, das nötig ist, um physiologische Eigenschaften eines Sportlers zu überwachen, in ein Kleidungsstück integriert. Des Weiteren, erfolgt die richtige Platzierung der Messvorrichtung durch korrektes Anlegen des Kleidungsstücks. Vorzugsweise bedeckt hierbei das Kleidungsstück den Oberkörper der zu überwachenden Person. Dabei kann es sich um einen Pullover, ein T-Shirt, ein Unterhemd oder um irgendein anderes Kleidungsstück handeln, das der Sportler während des Ausübens seiner Sportart trägt. Für die Messung mancher physiologischen Eigenschaften kann es allerdings vorteilhaft sein, die Messvorrichtung und/oder Sensorik in einem anderen Kleidungsstück zu integrieren, beispielsweise einer Hose oder einen Helm. Die Tatsache, dass der Sportler einfach nur ein Kleidungsstück anlegen muss, um physiologische Ereignisse oder Eigenschaften überwachen zu können, erhöht die Akzeptanz beim Sportler, da keine komplizierte Anbringung des Messsystems notwendig ist.

In vielen Fällen ist es notwendig zwischen Messsensoren und Messelektronik zu unterscheiden. Hierbei verarbeitet die Messelektronik physikalische Signale, die über die Messsensoren aufgenommen werden und physiologische Eigenschaften charakterisieren, weiter. Aus der Weiterverarbeitung resultieren dann die Messresultate, wie etwa Herzfrequenz oder Atemfrequenz. Oft können sowohl Messsensoren als auch Messelektronik in einer gemeinsam genutzten Vorrichtung untergebracht sein, die dann an einer bestimmten Stelle im Kleidungsstück integriert ist. In anderen Ausführungsformen ist es dagegen sinnvoll, die Messelektronik räumlich von den Sensoren zu trennen. So können hier die Sensoren an den für die Messung notwendigen Stellen im Kleidungsstück positioniert werden, während die Messelektronik, die meist sperriger ist als die Sensoren, an einer anderen Stelle in das Kleidungsstück integriert wird.

Ausführungsformen der Erfindung ermöglichen außerdem die Übertragung der Messergebnisse von physiologischen Eigenschaften während des Ausübens der Sportart. Hierzu wird zusätzlich zur Messelektronik und zu den Messsensoren eine Sendeeinheit in das Kleidungsstück integriert. In einigen Ausführungsformen der Erfindung nutzt die Sendeeinheit gemeinsam eine Vorrichtung mit den Messsensoren und der Messelektronik und ist somit an der gleichen Stelle im Kleidungsstück positioniert. Allerdings beeinflusst das Versenden von Signalen oft Messungen von physiologischen Eigenschaften. Demnach ist in anderen Ausführungsformen die Sendeeinheit von den Messsensoren räumlich getrennt und kann beispielsweise mit der Messelektronik eine gemeinsame Vorrichtung nutzen. In wieder anderen alternativen Ausführungsformen schließlich ist die Sendeeinheit räumlich sowohl von den Messsensoren als auch von der Messelektronik getrennt im Kleidungsstück untergebracht. In diesen Fällen kommuniziert die Messelektronik beispielsweise entweder über eine kabelgestützte Verbindung mit der Sendeeinheit oder kabellos mittels eines Nahbereichskommunikationsverfahrens, wie etwa Bluetooth.

In allen Ausführungsformen wird jedoch der Brustbereich des Kleidungsstücks von Messelektronik und Sendeeinheit frei gehalten. Des Weiteren werden die Messsensoren wenn möglich ebenfalls so platziert, dass der Brustbereich des Kleidungsstücks frei bleibt.

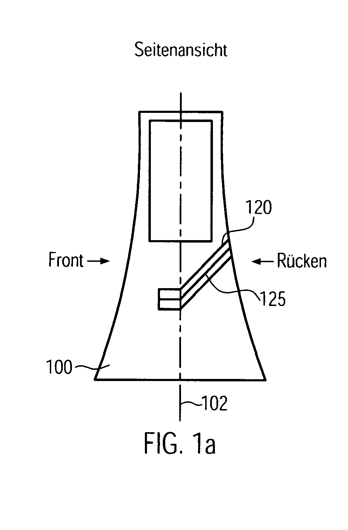

Fig. 1a bis 1c zeigen ein exemplarisches Kleidungsstück 100 zum Überwachen von physiologischen Eigenschaften. Hierbei sollen Prinzipien der vorliegenden Erfindung anhand der Messung der Herzfrequenz exemplarisch erläutert werden. Wie allerdings eingangs erwähnt, können auch andere physiologische Eigenschaften mittels geeigneter Sensorik und Messelektronik überwacht und ausgewertet werden.

Gemäß Fig. 1a wird in einer Ausführungsform die Vorrichtung zum Überwachen von physiologischen Eigenschaften in ein T-Shirt integriert, das vom Sportler während des Ausübens der Sportart getragen wird. Natürlich kann die Vorrichtung zum Überwachen von physiologischen Eigenschaften, wie oben ausgeführt, auch in anderen Kleidungsstücken, wie Pullovern, Unterhemden oder Tank Tops eingearbeitet sein.

Fig. 1a zeigt ein Kleidungsstück 100 zur Messung der Herzfrequenz des Trägers des Kleidungsstücks in einer Seitenansicht. Hilfslinie 102 teilt Kleidungsstück 100 in Vorderseite (Frontseite) und Rückseite. Gemäß einer Ausführungsform der vorliegenden Erfindung bilden Elektroden, die in Kontakt mit der Haut des Trägers des Kleidungsstücks stehen, die Messsensoren zur Überwachung der Herzfrequenz. In Fig. 1a ist exemplarisch eine Elektrode 120 gezeigt, die so in das Kleidungsstück eingearbeitet ist, dass sie sich im getragenen Zustand seitlich an den Rippen des Trägers beginnend in Richtung Wirbelsäule des Trägers zu den Schulterblättern fortsetzend erstreckt. Dabei ist die Elektrode in einer Ausführungsform so positioniert, dass im getragenen Zustand möglichst wenig Knochen unterhalb der Elektrode 120 liegt.

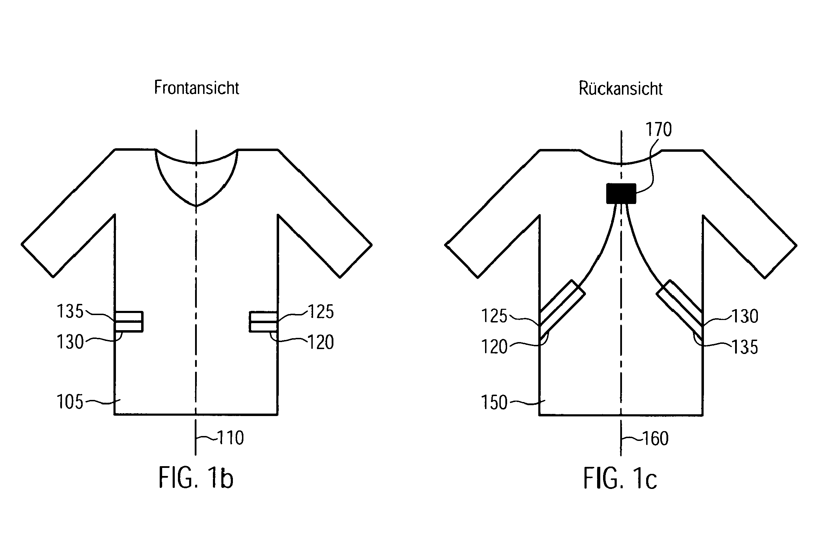

Um die Lage der Elektroden weiter zu verdeutlichen, geben in Fig. 1 b Linie 110 und in Fig. 1c Linie 160 die Lage der Wirbelsäule im getragenen Zustand des Kleidungsstücks 100 an. Dabei entspricht die Länge der Linie 110 beziehungsweise die Länge der Linie 160 nicht der Länge der Wirbelsäule. Tatsächlich sind die Linien 110 und 160 genau wie Linie 102 aus Fig. 1a nur als Hilfslinien zum besseren Verständnis der Erfindung gedacht.

Wie aus Fig. 1a bis 1c ersichtlich, ist die Elektrode 120 so positioniert, dass sie sich seitlich zwischen den fliehenden Rippen und den Rippenbogen des Trägers beginnend von der Vorderseite 105 des Kleidungsstücks 100 in Richtung der Rückseite 150 des Kleidungsstücks 100 bis zwischen die Schulterblätter des Trägers erstreckt. Im Besonderen kann die Elektrode 120 auf der Vorderseite 105 des Kleidungsstücks 100 zunächst waagrecht verlaufen, um dann auf der Rückseite 150 des Kleidungsstücks 100 linear in Richtung obere Brustwirbelsäule anzusteigen. Natürlich kann die Elektrode 120 auch auf andere Weise geformt sein. Besonders geeignet ist hierbei eine Form, die einem Zwischenrippenraum des Thorax des Trägers folgt. Hieraus ergibt sich auf der Rückseite 150 des Kleidungsstücks 100 eine geschwungene Form.

Wie aus Fig. 1b, die die Frontansicht des Kleidungsstücks 100 darstellt, und Fig. 1c, die die Rückansicht des Kleidungsstücks 100 zeigt, ersichtlich, ist symmetrisch zur Wirbelsäule des Trägers eine weitere Elektrode 130 im Kleidungsstück 100 integriert, die im Wesentlichen die gleichen Eigenschaften wie die Elektrode 120 aufweist. In einer Ausführungsform kann die Elektrode 130 allerdings auch entlang eines anderen Zwischenrippenraums des Trägers als die Elektrode 120 geführt werden. In diesen Ausführungsformen ist Elektrode 130 somit vertikal versetzt zu Elektrode 120.

Elektroden 120 und 130 bilden eine innere Oberfläche des Kleidungsstücks 100. In einer Ausführungsform sind sie aus leitfähigem Stretchstoff gebildet. Der Stretchstoff kann hierbei Lycra-ähnlich sein. Beispielsweise kann er zu etwa 92% aus silberbeschichtetem Nylon und zu etwa 8% aus Dorlastan bestehen. Der Oberflächenwiderstand eines solchen Stoffs ist im ungedehnten Zustand kleiner als 1Ω/sq. Die Dicke beträgt etwa 0,5 mm mit einem Gewicht von etwa 130 g/m².

In einigen Ausführungsformen sind die Elektroden 120 und 130 auf eine innere Oberfläche des Kleidungsstücks 100 aufgenäht oder aufgeklebt. Die Teile des Kleidungsstücks 100, auf die die Elektroden 120 und 130 aufgenäht oder aufgeklebt sind, können hierbei aus nichtleitfähigen Fasern bestehen, um die Elektroden nach außen hin zu isolieren. Allerdings können die Elektroden 120 und 130 auch selbst in das Kleidungsstück 100 eingenäht oder auf andere Weise integriert sein.

Wie weiter aus Fig. 1b bis 1c ersichtlich, ist jede Elektrode 120 und 130 mit einer leitfähigen Verbindung 125 beziehungsweise 135 in Kontakt. Hierbei sind die leitfähigen Verbindungen 125 und 135 entweder auf die Elektrode 120 beziehungsweise 130 der Länge nach aufgeklebt oder aufgestickt. In einigen Ausführungsformen sind die leitfähigen Verbindungen in den Stretchstoff, der die Elektroden bildet, eingewebt.

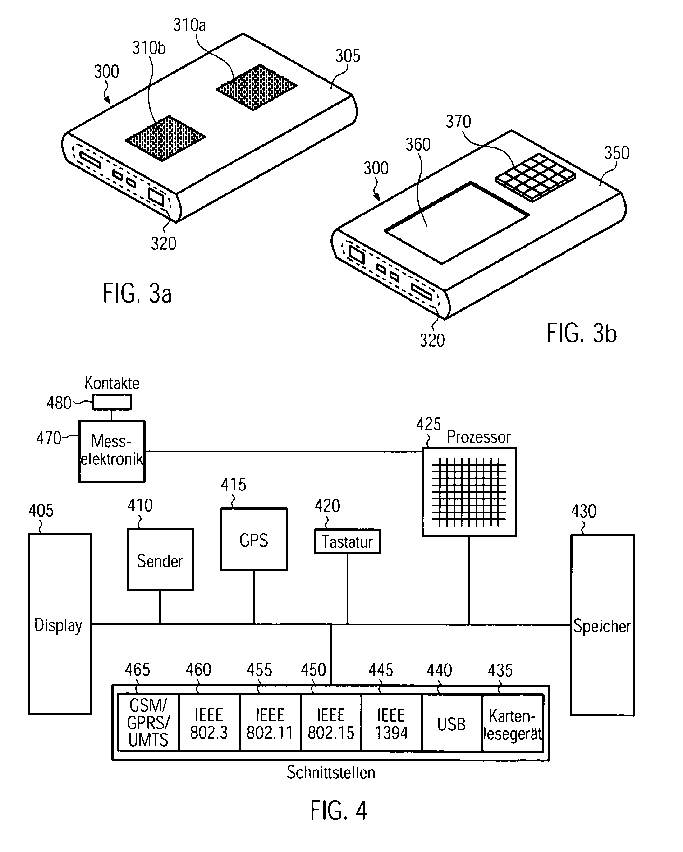

Von den Elektroden 120 oder 130 führen die leitfähigen Verbindungen 125 beziehungsweise 135 zu einer Vorrichtung 170 zum Überwachen physiologischer Eigenschaften, die genauer mit Bezug auf Fig. 3a und 3b sowie Fig. 4 beschrieben wird.

Die leitfähigen Verbindungen 125 und 135 können hierbei aus einem leitfähigen Faden oder Nähgarn bestehen, der oder das entweder in die nichtleitfähigen Fasern des Kleidungsstücks 100 eingewoben oder auf das Kleidungsstück 100 aufgenäht/aufgestickt oder aufgeklebt wird. Ein solcher Faden besteht beispielsweise aus silberbeschichtetem Nylon, dessen Widerstand kleiner als 5 Ω/cm ist.

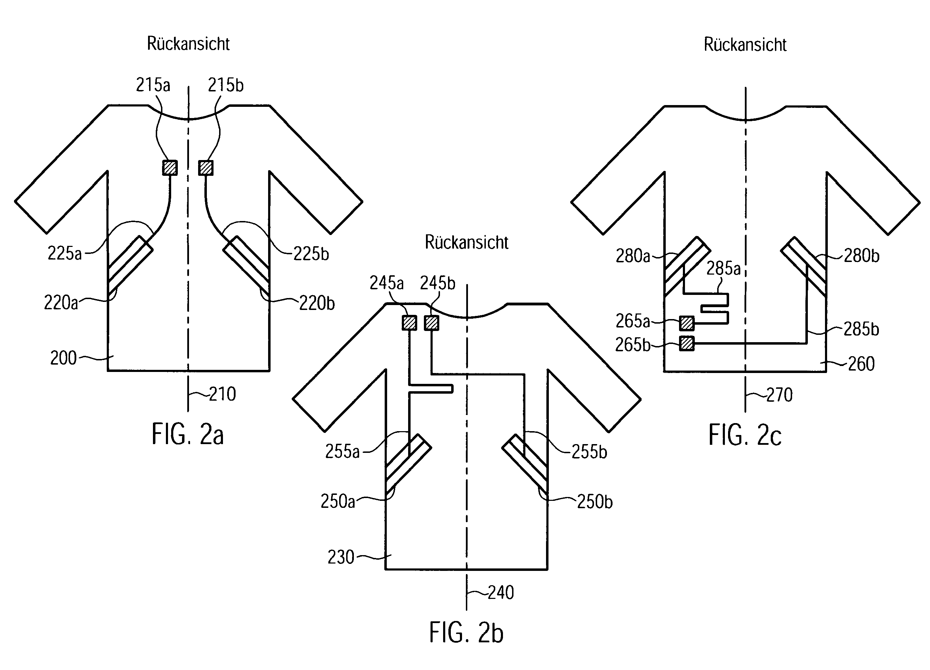

Die leitfähigen Verbindungen sind elektrisch leitend an die Vorrichtung 170 zum Überwachen physiologischer Eigenschaften gekoppelt. Wie oben beschrieben, und in weiteren Details unter Bezugnahmen auf Fig. 3a und 3b sowie Fig. 4 weiter unten erläutert, enthält die Vorrichtung 170 in einigen Ausführungsformen Messelektronik, die für eine Herzfrequenzmessung notwendig ist. In manchen Ausführungsformen ist die Vorrichtung 170 in das Kleidungsstück 100 fest integriert, in anderen Ausführungsformen ist sie vom Kleidungsstück 100 abnehmbar, wie unter Bezugnahme auf Fig. 2a bis 2c genauer erläutert. Bevorzugt ist die Vorrichtung 170 auf der Rückseite 150 des Kleidungsstücks 100 positioniert.

In manchen Ausführungsformen umfasst die Vorrichtung 170 auch eine Sendeeinheit, die die Ergebnisse der Messung von physiologischen Eigenschaften mittels einer kabellosen Übertragungstechnik an ein externes Empfangsgerät übermittelt. In anderen Ausführungsformen ist, wie oben erwähnt, die Sendeeinheit räumlich getrennt von der Vorrichtung 170, die die Messelektronik enthält, positioniert. So kann beispielsweise, die Messelektronik fest in das Kleidungsstück 100 integriert sein, während die Sendeeinheit abnehmbar ist oder umgekehrt.

Die Messelektronik in der Vorrichtung 170 ermittelt im Falle einer Herzfrequenzmessung elektrische Potenziale an den Elektroden 120 und 130. Da jeder Kontraktion des Herzmuskels eine elektrische Erregung vorausgeht, lassen sich durch Ableiten von elektrischen Potenzialen an der Körperoberfläche Rückschlüsse auf das Verhalten des Herzens im Allgemeinen und der Herzfrequenz im Besonderen schließen. Um den Einfluss der Zuleitungen 125 und 135 zu den Elektroden 120 und 130 zu minimieren, haben die leitfähigen Verbindungen in einigen Ausführungsformen den gleichen elektrischen Widerstand.

Natürlich können in Ausführungsformen der Erfindung auch mehr als zwei Elektroden zur Messung der Herzfrequenz verwendet werden. Hierbei können einzelne Elektroden entweder kurz geschlossen werden, beispielsweise mittels leitfähiger Verbindungen, oder jede einzelne Elektrode kann eine eigene leitfähige Verbindung zu der Vorrichtung 170 besitzen.

Fig. 2a bis 2c zeigen Ausführungsformen der Erfindung, in denen die Vorrichtung 170 zum Überwachen physiologischer Eigenschaften von der Rückseite 200, 230 oder 260 des Kleidungsstücks 100 abnehmbar ist. Ähnlich zu Hilfslinie 160 aus Fig. 1c geben hierbei Hilfslinie 210 in Fig. 2a, Hilfslinie 240 in Fig. 2b sowie Hilfslinie 270 in Fig. 2c die Lage der Wirbelsäule im getragenen Zustand des Kleidungsstücks an.

In diesen Ausführungsformen enden die leitenden Verbindungen 225a und 225b von Fig. 2a, 255a und 255b von Fig. 2b und 285a und 285b von Fig. 2c jeweils in den leitfähigen Kontakten 215a und 215b von Fig. 2a, 245a und 245b von Fig. 2b sowie 265a und 265b von Fig. 2c. Die leitfähigen Kontakte 215a/b, 245a/b, sowie 265a/b bilden hierbei eine Oberfläche des Kleidungsstücks 100. Natürlich ist die Anzahl der Kontakte nicht auf zwei beschränkt. So kann beispielsweise in Ausführungsformen, bei denen mehr als zwei Elektroden zur Messung der Herzfrequenz verwendet werden, jede Elektrode mit einem entsprechenden Kontakt leitfähig verbunden sein.

Die Elektroden 220a und 220b von Fig. 2a, Elektroden 250a und 250b von Fig. 2b sowie Elektroden 280a und 280b von Fig. 2c entsprechen hierbei den Elektroden 120 und 130 von Fig. 1a bis 1c. Ähnliches gilt für die leitfähigen Verbindungen 225a und 225b von Fig. 2a, Verbindungen 255a und 255b von Fig. 2b sowie Verbindungen 285a und 285b von Fig. 2c, die ähnlich zu den Verbindungen 125 und 135 aus Fig. 1a bis 1 c ausgebildet sind.

Die leitfähigen Kontakte 215a und 215b von Fig. 2a, 245a und 245b von Fig. 2b sowie 265a und 265b von Fig. 2c können in Ausführungsformen der Erfindung leitfähige Hacken-/Schlaufenbänder eines leitfähigen Klettverschlusses sein. In solchen Ausführungsformen besitzt die Vorrichtung 170 zum Überwachen physiologischer Eigenschaften die jeweiligen Gegenstücke der Hacken-/Schlaufenbänder an einer Oberfläche, wie in Fig. 3a genauer beschrieben. Die Klettverschlüsse verbinden dann die Elektroden zur Ableitung der elektrischen Potenziale an der Hautoberfläche mit der Messelektronik in der Vorrichtung 170 zur Messung der Herzfrequenz. Dabei ist der Widerstand des Hackenbandes etwa 1,8 Ω/sq und der Widerstand des Schlaufenbandes etwa 1,4 Ω/sq. Die erwartete Lebensdauer eines solchen Klettverschlusses liegt bei etwa 5000 Öffnungsprozessen.

Wie weiter oben beschrieben, kann in alternativen Ausführungsformen auch nur die Sende-/Speichereinheit für die Messresultate der Vorrichtung 170 trennbar mit den Kontakten an der Oberfläche verbindbar sein, wohingegen die Vorrichtung 170 mit der Messelektronik fest in das Kleidungsstück integriert ist. In solchen Fällen führen leitende Verbindungen, beispielsweise aus leitfähigem Garn wie oben beschrieben, von der Messelektronik zu den leitfähigen Kontakten, um die Sende-/Speichereinheit an die Vorrichtung 170 zu koppeln.

Fig. 2a zeigt ein Kleidungsstück bei dem die Vorrichtung 170 zum Überwachen physiologischer Eigenschaften abgenommen ist. Die beiden Kontakte 215a und 215b sind hierbei jeweils mit den Elektroden 220a und 220b mittels der leitfähigen Verbindungen 225a und 225b elektrisch gekoppelt. In dieser Ausführungsform sind die Kontakte 215a und 215b so auf einer äußeren Oberfläche der Rückseite 200 des Kleidungsstücks positioniert, dass sie sich, wenn das Kleidungsstück getragen wird, symmetrisch links und rechts der Wirbelsäule des Trägers befinden. Die Zuleitungen 225a und 225b sind in einigen Ausführungsformen so ausgebildet, dass sie den gleichen elektrischen Widerstand aufweisen.

In einer alternativen Ausführungsform, wie in Fig. 2b illustriert, sind die Kontakte 245a und 245b so auf einer äußeren Oberfläche der Rückseite 230 des Kleidungsstücks positioniert, dass sie sich, wenn das Kleidungsstück getragen wird, im Schulterbereich des Trägers befinden. Dabei können die Kontakte 245a und 245b im linken oder im rechten Schulterbereich des Kleidungsstücks integriert sein. Ein Vorteil dieser Ausführungsform gegenüber der Ausführungsform von Fig. 2a ist, dass die abnehmbare Vorrichtung 170 während des Tragens des Kleidungsstücks vom Träger selbst einfacher angebracht und abgenommen werden kann. Des Weiteren kommt die Vorrichtung 170 nicht auf der Wirbelsäule des Trägers zum Liegen, was das Verletzungsrisiko weiter minimiert.

Wie in Fig. 2b zu sehen, sind in diesen Ausführungsformen die elektrischen Widerstände der leitfähigen Verbindungen 255a und 255b aneinander angepasst, indem die Länge der Verbindung 255a der Länge der Verbindung 255b entspricht. Dies wird in einigen Ausführungsformen dadurch realisiert, dass die Länge der leitfähigen Verbindung 255a von der Elektrode 250a, die räumlich näher an den Kontakten 245a und 245b positioniert ist, durch Einführen von Schleifen verlängert wird, d.h. die Elektrode 250a nicht auf dem kürzesten Weg mit Kontakt 245a verbunden wird.

Eine weitere alternative Ausführungsform ist in Fig. 2c gezeigt. Hier sind die leitfähigen Kontakte 265a und 265b so auf einer äußeren Oberfläche der Rückseite 260 des Kleidungsstücks positioniert, dass sie, wenn das Kleidungsstück getragen wird, sich im seitlichen Lendenbereich des Trägers befinden. Ähnlich zu den Ausführungsformen der Fig. 2b können sich die Kontakte 265a und 265b entweder rechts oder links der Wirbelsäule des Trägers befinden. Auch hier wird in einigen Ausführungsformen die leitfähige Verbindung 285a von der Elektrode 280a, die räumlich näher bei den Kontakten 265a und 265b liegt, zum elektrischen Kontakt 265a nicht auf direktem Weg geführt, sondern so verlängert, dass der Widerstand der leitfähigen Verbindung 285a dem der leitfähigen Verbindung 285b entspricht.

Natürlich können die Kontakte 245a und 245b der Fig. 2b auch anders als abgebildet angeordnet sein. So können sie beispielsweise statt waagrecht, vertikal oder diagonal zueinander stehen. Ähnliches gilt für die Kontakte 265a und 265b der Fig. 2c. Die Kontakte 215a und 215b der Fig. 2a können auch vertikal angeordnet sein und entweder links oder rechts der Wirbelsäule des Trägers liegen. In manchen Ausführungsformen liegen die Kontakte 215a und 215b auch genau auf der Wirbelsäule des Trägers. Des Weiteren können Kontakte 245a und 245b auch auf einem Ärmel, falls vorhanden, auf der Rückseite 230 des Kleidungsstücks positioniert werden.

In alternativen Ausführungsformen sind die elektrischen Kontakte 215a, 215b, 245a, 245b, 265a und 265b nicht auf einer äußeren Oberfläche auf der Rückseite des Kleidungsstücks positioniert, sondern auf einer inneren Oberfläche der Rückseite des Kleidungsstücks. Dies hat den Vorteil, dass die Vorrichtung 170 zum Überwachen physiologischer Eigenschaften im angehefteten Zustand während des Ausübens der Sportart nicht so leicht versehentlich abgestreift werden kann, beispielsweise durch Berührung mit einem anderen Mitspieler oder mit dem Spielgerät. In einer bevorzugen Ausführungsform sind die Kontakte hier im Nackenbereich des Trägers positioniert.

Fig. 3a und 3b zeigen perspektivische Ansichten einer Vorrichtung 300 zum Überwachen physiologischer Eigenschaften. Dabei entspricht in manchen Ausführungsformen die Vorrichtung 170 zum Überwachen physiologischer Eigenschaften der Vorrichtung 300. Die Vorrichtung 300 enthält hierbei im Wesentlichen Messelektronik, die gemessene physikalische Signale weiterverarbeitet uns so als Messresultate physiologische Eigenschaften wie beispielsweise Herzfrequenz oder Atemfrequenz liefert. Wie aus Fig. 3a ersichtlich, befinden sich auf einer Oberfläche 305 der Vorrichtung 300 elektrisch leitfähige Kontakte 310a und 310b. Kontakte 310a und 310b werden mit Kontakten auf einer Oberfläche des Kleidungsstücks in Verbindung gebracht, um eine elektrische Verbindung zu den Messsensoren zu etablieren.

So sind in einigen Ausführungsformen Kontakte 310a und 310b die entsprechenden Gegenstücke zu den Kontakten 215a und 215b oder 245a und 245b oder 265a und 265b. Bei Ausführungsformen, bei denen die Kontakte des Kleidungsstücks als Hacken-/Schlaufenbänder eines Klettverschlusses ausgebildet sind, sind die Kontakte 310a und 310b die entsprechenden Gegenstücke des Klettverschlusses, wie oben beschrieben.

Des Weiteren kann sich auf einer Oberfläche der Vorrichtung 300 eine Reihe von Schnittstellenausgängen 320 befinden, wie beispielsweise ein USB-Ausgang, ein Firewire-Ausgang, ein Ethernet-Ausgang oder ein Flash-Karten-Laufwerk.

Fig. 3b zeigt eine perspektivische Ansicht der Vorrichtung 300, in der die der Oberfläche 305 gegenüberliegende Oberfläche 350 zu sehen ist. In manchen Ausführungsformen ist in dieser Oberfläche 350 ein Display 360 integriert, auf dem beispielsweise aktuelle Messwerte angezeigt werden können. Des Weiteren ist eine Tastatur 370 angebracht, so dass die Vorrichtung 300 direkt über Tastatur 370 beispielsweise mit Hilfe des Displays 360 bedient werden kann.

Fig. 4 zeigt eine schematische Ansicht der Vorrichtung 300 gemäß einer exemplarischen Ausführungsform. Die einzelnen Module der Vorrichtung 300 können hierbei über einen Bus 400 miteinander verbunden sein. Die Hauptaufgabe des Prozessors 425 liegt darin, die von den Messsensoren über Kontakte 480 (entsprechen Kontakte 310a und 310b aus Fig. 3a) empfangenen physikalischen Signale zu verarbeiten und auszuwerten. Dabei ist der Prozessor an eine Messelektronik 470 gekoppelt, die eingerichtet ist, die physikalischen Signale, wie etwa elektrische Potenziale im Falle einer Herzfrequenzmessung, zu detektieren. Der Prozessor 425 leitet dann die Auswertung der Messsignale weiter zum Speicher 430 und/oder zur Sendeeinheit 410.

Wie oben mehrfach beschrieben, kann die Sendeeinheit 410 alternativ auch in einer externen Vorrichtung untergebracht sein. In allen Fällen versendet die Sendeeinheit 410 die Messresultate über Funk zu einem externen Empfänger. In einigen Ausführungsformen verfügt die Vorrichtung 300 auch über ein GSM/GPRS/UMTS-Modul 465, das beispielsweise im Sender 410 integriert ist. In solchen Ausführungsformen können die Messresultate auch über ein Telekommunikationsnetzwerk versandt werden. Verfügt die Vorrichtung 300 über ein Wi-fi-Modul 455, welches ebenfalls in der Sendeeinheit 410 integriert sein kann, so können die Messresultate auch über ein WLAN an einen externen Empfänger verschickt werden.

Der Prozessor 425 ist ebenfalls eingerichtet, die Messresultate im Speicher 430 zu hinterlegen. Von dort können dann die Messresultate mittels der USB-Schnittstelle 440, der Firewire-Schnittstelle 445, der Bluetooth-Schnittstelle 450, der Ethernet-Schnittstelle 460 oder des Flash-Karten-Laufwerk 435 an einen externen Empfänger übertragen werden. Alternativ kann man auch mittels Befehlseingabe über die Tastatur 420 die Messresultate am Display 405 anzeigen lassen.

In einigen Ausführungsformen verfügt die Vorrichtung 300 ebenfalls über einen GPS-Empfänger 415. Mittels des GPS-Empfängers 415 lässt sich die Position des Spielers während des Trainings ermitteln. Die GPS-Koordinaten können dann zusammen mit den gemessenen physiologischen Eigenschaften im Speicher abgelegt werden und/oder direkt zu einem externen Empfänger mittels Sender 410 und/oder Module 465 oder 455 versandt werden. So können beispielsweise Rückschlüsse auf das Laufpensum während eines Spiels geschlossen werden und so mit Hilfe einer Herzfrequenzmessung die Fitness des Sportlers abgeschätzt werden. Es lassen sich beispielsweise auch genaue Aussagen über das Tempo des Spielers und der dazugehörigen Herzfrequenz (oder anderer gemessener physiologischer Eigenschaften) ermitteln.

Die Stromversorgung der Vorrichtung 300 kann hierbei über Batterien sichergestellt werden.

So bietet die vorliegende Erfindung in ihren Ausführungsformen zahlreiche Möglichkeiten zur Überwachung physiologischer Eigenschaften eines Sportlers während der Ausübung seiner Sportart, ohne den Sportler zu behindern oder das Verletzungsrisiko zu erhöhen. Des Weiteren wird die Gefahr einer Beschädigung der Messsensoren und der Messelektronik vermindert.

## Patentansprüche

1. Kleidungsstück (100) umfassend eine Vorrichtung zum Überwachen (170; 300) physiologischer Eigenschaften eines Trägers des Kleidungsstücks, wobei die Vorrichtung zum Überwachen auf der Rückseite (150; 200; 230; 260) des Kleidungsstücks positioniert ist.

2. Kleidungsstück nach Anspruch 1, wobei die Vorrichtung zum Überwachen des Weiteren eingerichtet ist, physikalische Signale, die die zu überwachenden physiologischen Eigenschaften des Trägers des Kleidungsstücks charakterisieren, zu empfangen und weiterzuverarbeiten.

3. Kleidungsstück nach Anspruch 2, wobei die Vorrichtung zum Überwachen des Weiteren eingerichtet ist, Messresultate, die aus der Weiterverarbeitung der physikalischen Signale resultieren, zu speichern und zu versenden.

4. Kleidungsstück nach Anspruch 3, wobei das Kleidungsstück des Weiteren umfasst:
zwei leitfähige Elektroden; und
leitfähige Verbindungen von den leitfähigen Elektroden zu der Vorrichtung zum Überwachen.

5. Kleidungsstück nach Anspruch 4, wobei die Vorrichtung zum Überwachen trennbar mit dem Kleidungsstück verbunden ist.

6. Kleidungsstück nach Anspruch 5, wobei:
die Vorrichtung zum Überwachen leitfähige Kontakte an einer Oberfläche umfasst;
das Kleidungsstück leitfähige Kontakte an einer Oberflache der Rückseite umfasst; und
die leitfähigen Kontakte der Vorrichtung zum Überwachen mit den leitfähigen Kontakten des Kleidungsstücks trennbar verbindbar sind.

7. Kleidungsstück nach Anspruch 6, wobei das Kleidungsstück des Weiteren umfasst:
leitfähige Verbindungen von den leitfähigen Elektroden zu den leitfähigen Kontakten auf der Oberfläche des Kleidungsstücks.

8. Kleidungsstück nach Anspruch 7, wobei:
die leitfähigen Elektroden aus leitfähigem Stretchstoff sind;
die leitfähigen Verbindungen von den leitfähigen Elektroden zu den leitfähigen Kontakten aus leitfähigem Nähgarn sind;
die leitfähigen Kontakte an der Oberfläche der Rückseite des Kleidungsstücks und die leitfähigen Kontakte an der Oberfläche der Vorrichtung zum Überwachen leitfähige Klettverschlüsse sind; und
das Kleidungsstück des Weiteren nichtleitfähige Fasern umfasst und die leitfähigen Verbindungen von den leitfähigen Elektroden zu den leitfähigen Kontakten auf der Oberfläche des Kleidungsstücks in die nichtleitfähigen Fasern des Kleidungsstücks eingewoben sind, oder zumindest ein Teil des Kleidungsstücks aus nichtleitfähiger Faser hergestellt ist und die leitfähigen Verbindungen von den leitfähigen Elektroden zu den leitfähigen Kontakten auf der Oberfläche des Kleidungsstücks auf den Teil des Kleidungsstücks, der aus nichtleitfähiger Faser hergestellt ist, geklebt oder aufgestickt sind.

9. Kleidungsstück nach einem der Ansprüche 7 bis 8, wobei:
die leitfähigen Elektroden eine innere Oberfläche des Kleidungsstücks bilden;
die beiden Elektroden sich symmetrisch zur Wirbelsäule des Trägers befinden, wenn das Kleidungsstück getragen wird; und
die beiden Elektroden sich seitlich zwischen den fliehenden Rippen und den Rippenbogen des Trägers beginnend von der Vorderseite des Kleidungsstücks in Richtung der Rückseite des Kleidungsstücks bis zwischen die Schulterblätter des Trägers erstrecken, wenn das Kleidungsstück getragen wird.

10. Kleidungsstück nach einem der Ansprüche 7 bis 9, wobei die Vorrichtung zum Überwachen des Weiteren einen Prozessor, einen Speicher, eine USB-Schnittstelle, eine Firewire-Schnittstelle, ein Wi-fi-Modul, eine Bluetooth-Schnittstelle, ein GSM-Modul, ein GPRS Modul, ein UMTS Modul oder ein GPS-Modul umfasst.

11. Kleidungsstück nach einem der Ansprüche 7 bis 10, wobei die zu überwachende physiologische Eigenschaft die Herzfrequenz des Trägers ist und die Vorrichtung zum Überwachen eingerichtet ist, elektrische Potenziale an den Elektroden zu messen.

12. Kleidungsstück nach einem der Ansprüche 7 bis 11, wobei:
die leitfähigen Kontakte des Kleidungsstücks an einer äußeren Oberfläche auf der Rückseite des Kleidungsstücks angebracht sind; und
die leitfähigen Kontakte des Kleidungsstücks sich im getragenen Zustand entweder rechts oder links der Wirbelsäule des Trägers befinden.

13. Kleidungsstück nach Anspruch 12, wobei:
die leitfähigen Kontakte des Kleidungsstücks im getragenen Zustand sich im Schulterbereich des Trägers befinden oder die leitfähigen Kontakte des Kleidungsstücks sich im getragenen Zustand im Lendenbereich des Trägers befinden; und
die leitfähigen Verbindungen von den leitfähigen Elektroden zu den leitfähigen Kontakten den gleichen elektrischen Widerstand haben.

14. Kleidungsstück nach einem der Ansrpüche 7 bis 11, wobei:
die leitfähigen Kontakte an einer äußeren Oberfläche auf der Rückseite des Kleidungsstücks angebracht sind; und
die leitfähigen Kontakte des Kleidungsstücks sich im getragenen Zustand symmetrisch rechts und links der Wirbelsäule des Trägers befinden.

15. Kleidungsstück nach einem der Ansprüche 7 bis 11, wobei:
die leitfähigen Kontakte an einer inneren Oberfläche auf der Rückseite des Kleidungsstücks angebracht sind; und
die leitfähigen Kontakte des Kleidungsstücks sich im getragenen Zustand im Nackenbereich des Trägers befinden.
